(19) Europäisches Patentamt
European Patent Office
Office européen des brevets

(11) EP 0 728 745 A1

(12) EUROPÄISCHE PATENTANMELDUNG

(43) Veröffentlichungstag: **28.08.1996 Patentblatt 1996/35**

(21) Anmeldenummer: **96101796.9**

(22) Anmeldetag: **08.02.1996**

(51) Int. Cl.$^6$: **C07D 221/14**, C08F 255/02, C07D 471/06 // (C07D471/06, 235:00, 221:00)

(84) Benannte Vertragsstaaten: **AT BE CH DE FR GB IT LI NL**

(30) Priorität: **21.02.1995 DE 19505941**

(71) Anmelder: **BAYER AG**
**51368 Leverkusen (DE)**

(72) Erfinder:
- **Chen, Yun, Dr.**
  **D-47800 Krefeld (DE)**
- **Köhler, Burkhard, Dr.**
  **D-51373 Leverkusen (DE)**
- **Wehrmann, Rolf, Dr.**
  **D-47800 Krefeld (DE)**

# (54) 1,8-Naphthalimid-Derivate, Verfahren zur Herstellung und ihre Verwendung als Zwischenprodukte

(57) 1,8-Naphthalimid-Derivate der Formeln (Ia), (Ib) und (Ic)

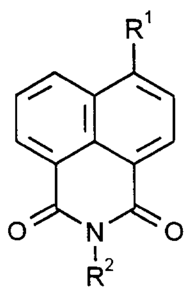

(Ia)

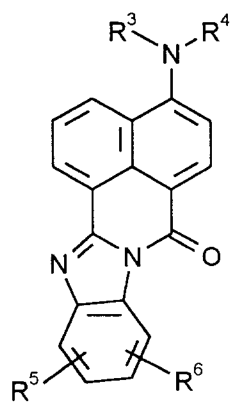

(Ib)

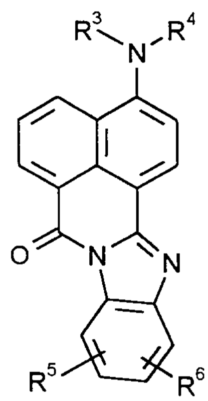

(Ic)

Verfahren zu deren Herstellung sowie deren Verwendung als Zwischenprodukte zur Herstellung von (Co)Polymeren mit Luminophoren an den Seitenketten.

EP 0 728 745 A1

## Beschreibung

Die Erfindung betrifft 1,8-Naphthalimid-Derivate der Formel (Ia), (Ib) und (Ic)

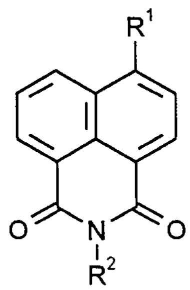
(Ia)

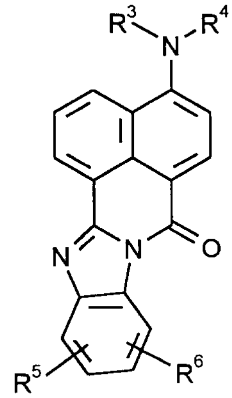
(Ib)

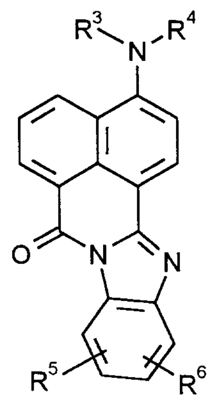
(Ic)

worin

$R^1$ für Wasserstoff, Halogen, Nitro, $C_1$-$C_4$-Alkoxycarbonyl, $C_1$-$C_4$-Acyl, $C_8$-$C_{24}$-Aralkenyl, unsubstituiertes Amino oder für ein- oder zweifach, gleich oder verschieden durch $C_1$-$C_{30}$-Alkyl, $C_6$-$C_{18}$-Aryl, $C_7$-$C_{24}$-Aralkyl substituiertes Amino steht, wobei die oben genannten Kohlenstoffketten selbst durch Hydroxy und/oder Carboxy substituiert sein können,

$R^1$ weiterhin für Morpholinyl, Piperidinyl, Pyrrolidinyl oder Piperazinyl steht, die einen oder zwei Substituenten ausgewählt aus Hydroxy, Methyl, Ethyl und/oder Phenyl tragen können,

$R^2$ für Wasserstoff oder $C_1$-$C_{30}$-Alkyl, $C_6$-$C_{18}$-Aryl, $C_7$-$C_{24}$-Aralkyl steht, welche ein- oder mehrfach durch Hydroxy und/oder Carboxy substituiert sein können,
und mindestens einer der Reste $R^1$ oder $R^2$ über eine Hydroxy- oder Carboxygruppe verfügt,

$R^3$ und $R^4$ unabhängig voneinander für $C_1$-$C_{30}$-Alkyl, $C_6$-$C_{18}$-Aryl, $C_7$-$C_{24}$-Aralkyl stehen, die durch Hydroxy und/oder Carboxy substituiert sein können, wobei mindestens einer der Reste $R^3$ oder $R^4$ über eine Hydroxy- oder Carboxygruppe verfügt.

$R^3$ und $R^4$ weiterhin gemeinsam mit dem Stickstoffatom, an das sie gebunden sind, für Morpholinyl, Piperidyl, Pyrolidyl oder Piperazinyl stehen, die einen oder zwei gleich oder verschiedene Substituenten ausgewählt aus Methyl, Ethyl und Phenyl tragen können und über mindestens eine Hydroxy- oder Carboxygruppe verfügen,

$R^5$ und $R^6$ unabhängig voneinander Wasserstoff, Halogen, Nitro, $C_1$-$C_{30}$-Alkyl, $C_1$-$C_{30}$-Alkoxy, $C_6$-$C_{18}$-Aryl, $C_7$-$C_{24}$-Aralkyl, $C_1$-$C_4$-Alkoxycarbonyl, $C_1$-$C_4$-Acyl oder Di($C_1$-$C_6$-Alkyl)amino bedeuten, wobei die aliphatischen Kohlenstoffketten, wie z.B. Alkyl, Alkoxy, Alkylamino, Aralkyl, in $R^1$, $R^2$, $R^3$ und $R^4$ durch eines oder mehrere, vorzugsweise ein oder zwei Heteroatome ausgewählt aus Sauerstoff, Stickstoff und Schwefel und/oder durch einen oder mehrere, vorzugsweise ein oder zwei Phenylenringe, die durch $C_1$-$C_4$-Alkyl und/oder Halogen substituiert sein können, unterbrochen sein können.

Die erfindungsgemäßen 1,8-Naphthalimid-Derivate der Formeln (Ia), (Ib) und (Ic), tragen mindestens eine Hydroxy- oder eine Carboxygruppe, über die sie an Polymerseitenketten chemisch gebunden werden können. Daher eignen sie sich zur Herstellung von Polymeren mit Luminophoren an den Seitenketten. Solche polymeren Stoffe können in der lichtemittierenden Schicht einer Leuchtdiode eingesetzt werden.

In der oben genannten Formel (Ia) steht

$R^1$ vorzugsweise für Wasserstoff, Chlor, Brom, Nitro, Methoxycarbonyl, Ethoxycarbonyl, n- oder iso-Propoxycarbonyl, Methylcarbonyl, Ethylcarbonyl, n- oder iso-Propylcarbonyl, Amino, für Amino, welches ein- oder zweifach, gleich oder verschieden durch $C_1$-$C_{15}$-Alkyl, jeweils gegebenenfalls durch Methyl und/oder Ethyl substituiertes Phenyl, Naphthyl, Phenyl-$C_1$-$C_4$-alkyl oder Naphthyl-$C_1$-$C_4$-alkyl substituiert ist, wobei die oben genannten Kohlenstoffketten selbst durch Hydroxy und/oder Carboxy substituiert sein können, $R^1$ weiterhin vorzugsweise für Morpholinyl, Piperidinyl, Pyrrolidinyl oder Piperazinyl steht, die einen oder zwei Substituenten ausgewählt aus Methyl, Ethyl und/oder Phenyl tragen können.

$R^2$ steht vorzugsweise für $C_1$-$C_{15}$-Alkyl, Phenyl oder Phenyl-$C_1$-$C_6$-alkyl, welche durch Hydroxy und/oder Carboxy und die aromatischen Ringen zusätzlich noch durch Halogen, $C_1$-$C_6$-Alkyl oder $C_1$-$C_6$-Alkoxy substituiert sein können.

Mindestens einer der Reste $R^1$ und $R^2$ muß über eine Hydroxy- oder Carboxygruppe verfügen.

$R^1$ steht insbesondere für Chlor, Brom, Amino, welches ein- oder zweifach, gleich oder verschieden durch $C_1$-$C_{15}$-Alkyl substituiert ist, Morpholinyl, Piperidinyl, Pyrrolidinyl oder Piperazinyl, wobei die oben genannten Kohlenstoffketten selbst durch Hydroxy oder Carboxy substituiert sein können.

$R^2$ steht insbesondere für $C_1$-$C_{12}$-Alkyl, gegebenenfalls durch Halogen, $C_1$-$C_6$-Alkyl, $C_1$-$C_6$-Alkoxy substituiertes Phenyl, die eine Hydroxy- oder Carboxygruppe tragen können.

$R^3$ und $R^4$ in den Formeln (Ib) und (Ic) stehen vorzugsweise unabhängig voneinander für $C_1$-$C_{15}$-Alkyl, Phenyl, Phenyl-$C_1$-$C_6$-alkyl, Naphthyl, Naphthyl-$C_1$-$C_6$-alkyl, die einfach oder mehrfach, insbesondere einfach durch Hydroxy oder Carboxy substituiert sein können, wobei mindestens einer der Reste $R^3$ oder $R^4$ über eine Hydroxy- oder Carboxygruppe verfügt.

$R^3$ und $R^4$ stehen weiterhin gemeinsam mit dem Stickstoffatom, an das sie gebunden sind, vorzugsweise für Piperidinyl oder Piperazinyl, die einen oder zwei gleich oder verschiedene Substituenten ausgewählt aus Methyl, Ethyl und Phenyl tragen können und über mindestens eine Hydroxy- oder Carboxygruppe verfügen,

$R^5$ und $R^6$ in den Formeln (Ib) und (Ic) stehen vorzugsweise unabhängig voneinander für Wasserstoff, Halogen, $C_1$-$C_{15}$-Alkyl, $C_1$-$C_{15}$-Alkoxy, $C_1$-$C_4$-Alkoxycarbonyl, $C_1$-$C_4$-Acyl- oder Di($C_1$-$C_6$-Alkyl)amino, jeweils durch Methyl und/oder Ethyl substituiertes Phenyl, Phenyl-$C_1$-$C_6$-alkyl, Naphthyl oder Naphthyl-$C_1$-$C_6$-alkyl.

$R^3$ und $R^4$ stehen insbesondere für $C_1$-$C_{12}$-Alkyl, Phenyl, Phenyl-$C_1$-$C_6$-alkyl, die durch eine Hydroxy- oder Carboxygruppe substituiert sein können.

$R^5$ und $R^6$ stehen insbesondere für Wasserstoff, Halogen, $C_1$-$C_{12}$-Alkyl, $C_1$-$C_{12}$-Alkoxy, Di($C_1$-$C_6$-alkyl)amino, Phenyl.

Auch in den bevorzugten und besonders bevorzugten Definitionen von $R^1$, $R^2$, $R^3$ und $R^4$ können die aliphatischen Kohlenstoffketten wie oben angegeben unterbrochen sein.

Die Zahl der Hydroxy- und/oder Carboxygruppen beträgt mindestens eine, es können jedoch auch bis zu vier Hydroxy- und/oder Carboxygruppen sein.

Die aromatischen Ringe in den oben genannten Resten können einfach bis fünffach, vorzugsweise einfach bis dreifach, gleich oder verschieden durch die genannten Substituenten substituiert sein.

Die Erfindung betrifft weiterhin ein Verfahren zur Herstellung von 1,8-Naphthalimid-Derivaten der Formel (Ia)

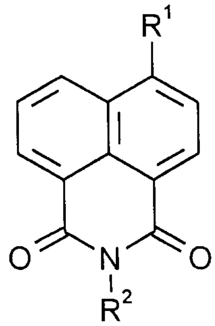

(Ia)

worin
$R^1$ und $R^2$ die obengenannte Bedeutung haben, dadurch gekennzeichnet, daß man entweder

a) ein 1,8-Naphthalsäureanhydrid der Formel (II) und ein primäres Amin der Formel (III),

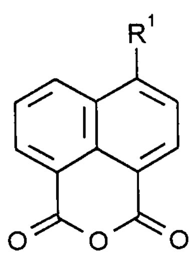

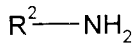

(II) (III)

bei 50 bis 250°C, vorzugsweise 90 bis 140°C, gegebenenfalls in Gegenwart von Verdünnungsmitteln wie beispielsweise Essigsäure, Butanol, Chlorbenzol, Toluol oder Xylol miteinander umsetzt oder

b) im Falle, daß $R^1$ in Formel (I) ein- oder zweifach substituiertes Amino oder cyclisches Amino bedeutet, ein 1,8-Naphthalimid der Formel (Id)

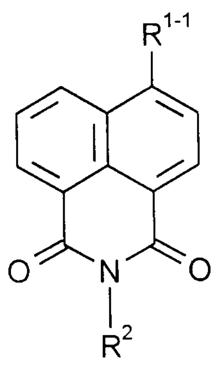

(Id)

worin

$R^{1-1}$ für Halogen, vorzugsweise Chlor, Brom oder Iod, oder Nitro steht,

welches aus einem 1,8-Naphthalsäureanhydrid der Formel (IIa) und einem primären Amin der Formel (III)

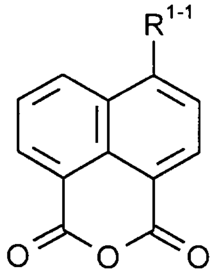

(IIa)

$R^2$-$NH_2$

(III)

worin $R^{1-1}$ und $R^2$ die oben genannte Bedeutung haben,
bei Temperaturen von 50 bis 250°C, vorzugsweise 90 bis 140°C, gegebenenfalls in Gegenwart von Verdünnungsmitteln wie beispielsweise Essigsäure, Butanol, Chlorbenzol, Toluol oder Xylol hergestellt wird,
und die so erhaltene Verbindung der Formel (Id) anschließend mit einem primären oder sekundären Amin, Piperidin, Morpholin, Pyrolidin oder Piperazin gemäß der Definition von $R^1$, oder einer wäßrigen Ammoniak-Lösung gegebenenfalls in Gegenwart von Lösungsmitteln wie beispielsweise Methoxyethanol oder Butanol gegebenenfalls unter Katalyse von beispielsweise einem Kupfer(II)-Salz bei Temperaturen von 50 bis 250°C, vorzugsweise 100 bis 150°C umsetzt.

Die Erfindung betrifft weiterhin ein Verfahren zur Herstellung von 1,8-Naphthalimid-Derivaten der Formel (Ib) und (Ic) (Verfahren c),

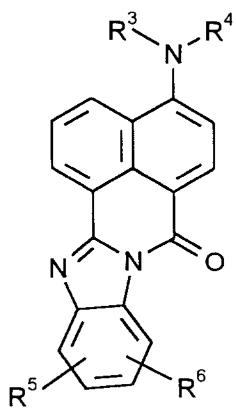

(Ib)

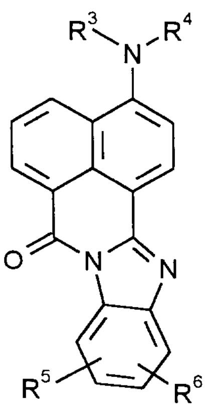

(Ic)

worin
$R^3$, $R^4$, $R^5$ und $R^6$ die obengenannte Bedeutung haben,
wobei man ein 1,8-Naphthalimid-Derivat der Formel (IV),

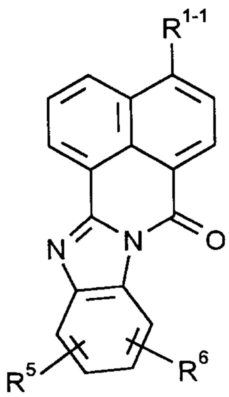 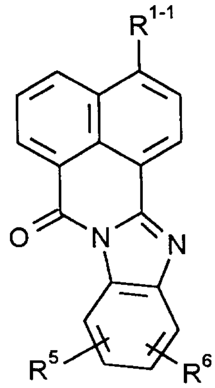

(IVa) (IVb)

worin
$R^5$, $R^6$ und $R^{1-1}$ den obigen Bedeutungsumfang haben,
aus einem 1,8-Naphthalsäureanhydrid der Formel (IIa)

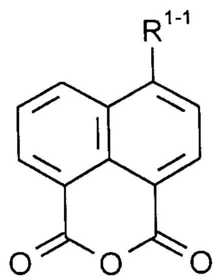

(IIa)

worin $R^{1-1}$ die oben genannte Bedeutung hat,
und einem o-Phenylendiamin der Formel (V)

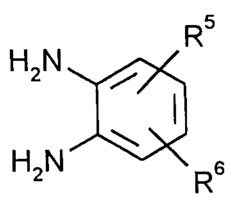

(V)

worin $R^5$ und $R^6$ die oben genannten Bedeutungen haben,
bei Temperaturen von 50 bis 250°C, vorzugsweise 90 bis 140°C, gegebenenfalls in Gegenwart von Lösungsmitteln wie beispielsweise Essigsäure, Butanol, Chlorbenzol, Toluol oder Xylol herstellt und das 1,8-Naphthalimid-Derivat der Formel (IV) anschließend mit einem sekundären Amin der Formel (VI),

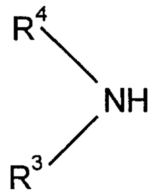

(VI)

worin $R^3$ und $R^4$ die oben genannte Bedeutung haben,
unter Katalyse von beispielsweise einem Kupfer(II)-Salz bei Temperaturen von 50 bis 250°C, vorzugsweise 100 bis 150°C, gegebenenfalls in Gegenwart eines Lösungsmittels wie beispielsweise Methoxyethanol oder Butanol, umsetzt.

Bei der Durchführung des erfindungsgemäßen Verfahrens a) zur Herstellung der 1,8-Naphthalimid-Derivate der Formel (Ia) setzt man im allgemeinen pro Mol Verbindung der Formel (II) 1 bis 1,8 Mol, vorzugsweise 1,2 bis 1,4 Mol primäres Amin der Formel (III) ein.

Bei der Durchführung des erfindungsgemäßen Verfahrens b) zur Herstellung der 1,8-Naphthalimid-Derivate der Formel (I) setzt man im allgemeinen pro Mol Verbindung der Formel (IIa) 1 bis 1,8 Mol, vorzugsweise 1,2 bis 1,4 Mol des primären Amins der Formel (III) und pro Mol Verbindung der Formel (Id) 1,2 bis 5 Mol, vorzugsweise 2 bis 2,5 Mol des entsprechenden unsubstituierten, primären, sekundären oder cyclischen Amins.

Die Herstellung der erfindungsgemäßen 1,8-Naphthalimid-Derivate der Formel (I), Verfahren (a) und (b) wird beispielhalft durch das folgende Formelschema dargestellt:

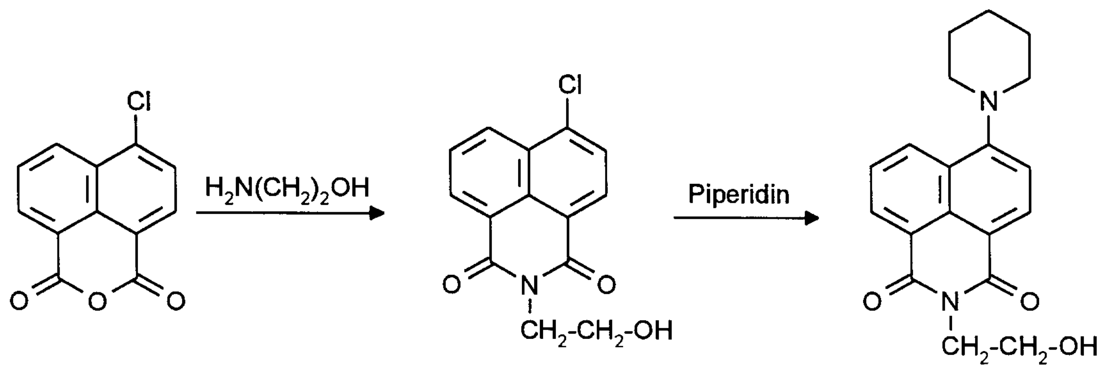


Hierbei wird zunächst das 4-Chlor-N-hydroxyethyl-1,8-naphthalimid durch Umsetzung von 4-Chlor-naphthalsäureanhydrid und 2-Aminoethanol hergestellt. Anschließend versetzt man das 4-Chlor-N-hydroxyethyl-1,8-naphthalimid mit Piperidin in Gegenwart katalytischer Menge von einem Kupfer(II)-Salz unter Bildung des erwünschten N-Hydroxyethyl-4-piperidino-1,8-naphthalimids.

Bei der Durchführung des erfindungsgemäßen Verfahrens c) zur Herstellung der 1,8-Naphthalimid-Derivate der Formel (Ib) und (Ic) setzt man im allgemeinen pro Mol Verbindung der Formel (IIa) 1 bis 1,8 Mol, vorzugsweise 1,2 bis 1,4 Mol des o-Phenylendiamins der Formel (V) und pro Mol Verbindung (IVa-b) 1,2 bis 5 Mol, vorzugsweise 2 bis 2,5 Mol des sekundären Amins der Formel (VI) ein.

Die Herstellung der erfindungsgemäßen 1,8-Naphthalimid-Derivate der Formel (Ib und c) wird beispielhaft durch das folgende Formelschema dargestellt:

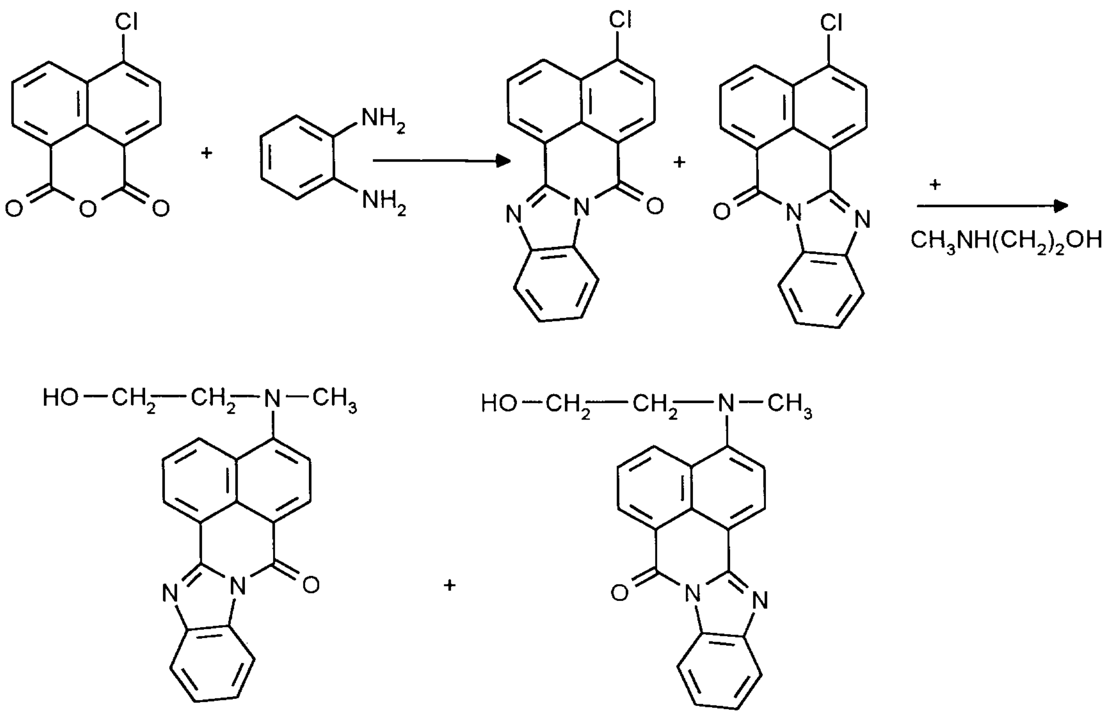


Hierbei wird zunächst das 4/5-Chlor-1,8-naphthoylen-1',2'-benzimidazol, welches als ein Isomerengemisch (ca. 3:1) anfällt, durch Umsetzung von 4-Chlornaphthalsäureanhydrid und o-Phenylendiamin hergestellt. Anschließend versetzt man das 4/5-Chlor-1,8-naphthoylen-1',2'-benzimidazol mit 2-(Methylamino)-ethanol in Gegenwart katalytischer Menge von einem Kupfer(II)-Salz unter Bildung des erwünschten 4/5-(N-Methyl-N-hydroxyethyl)amino-1,8-naphthoylen-1',2'-benzimidazols.

Die Ausgangsprodukte der Formeln (II), (III), (V) und (VI) zur Herstellung der erfindungsgemäßen Verbindungen der Formel (Ia), (Ib) und (Ic) sind allgemein bekannte Verbindungen der organischen Chemie.

Die erfindungsgemäßen Verbindungen der Formeln (Ia), (Ib) und (Ic) eignen sich zur Herstellung von (Co)Polymeren mit Luminophoren an den Seitenketten, die zum Aufbau von elektrolumineszierenden Anordnungen verwendet werden können. Die (Co)Polymere sind aufgebaut aus mindestens einem wiederkehrenden Baustein der allgemeinen Formel (1) oder (2) und gegebenenfalls (3)

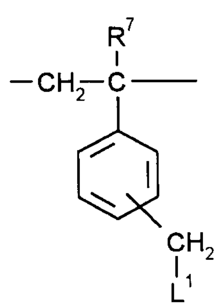
(1)

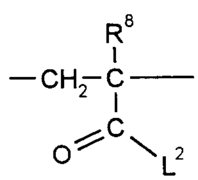
(2)

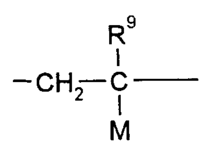
(3)

worin
$R^7$, $R^8$ und $R^9$ unabhängig voneinander Wasserstoff oder $C_1$-$C_6$-Alkyl bedeuten,

M für CN oder $C_1$-$C_{30}$-Alkoxycarbonyl, $C_1$-$C_{30}$-(Di)Alkylaminocarbonyl, $C_1$-$C_{30}$-Alkylcarbonyl steht, die jeweils durch Hydroxy oder $C_1$-$C_6$-Alkoxycarbonyl substituiert sein können, weiterhin für Phenyl, Naphthyl, Anthracenyl, Pyridyl oder Carbazolyl steht, die jeweils durch Reste aus der Gruppe Halogen, Hydroxy, Silyl, $C_1$-$C_{30}$-Alkyl, $C_6$-$C_{18}$-Aryl, $C_1$-$C_{30}$-Alkoxy, $C_1$-$C_{30}$-Alkoxycarbonyl, $C_1$-$C_{30}$-Acyloxy und $C_1$-$C_{30}$-Alkylcarbonyl substituiert sein können,

$L^1$ und $L^2$ unabhängig voneinander für oben beschriebene Verbindungen der Formeln (Ia), (Ib) oder (Ic) steht, wobei die Verknüpfung an die Methylengruppe am Phenyl des Monomer (1) bzw. an die Carbonylgruppe des Monomer (2) über das Sauerstoff-Atom der Hydroxy- oder Carboxygruppe und gegebenenfalls über den Stickstoff aus der Amino-Gruppe mit einer reaktiven Gruppe, z.B. Halogen, erfolgt. Bei Monomer (1) ist die reaktive Gruppe z.B. $-CH_2Cl$, bei Monomer (2)-CO-Cl.

Der Anteil der Struktureinheiten der Formel (3) beträgt 0 bis 99,5, vorzugsweise 40 bis 99,5 Mol-% und der Anteil an Struktureinheiten (1) und/oder (2) jeweils 0,5 bis 100, vorzugsweise 0,5 bis 60 Mol-%, wobei sich die Molanteile zu 100 % ergänzen.

Die oben beschriebenen (Co)Polymere lassen sich durch übliche Polymerisationsverfahren aus den entsprechenden Monomeren der Formeln (4) und (5) und gegebenenfalls (6) herstellen:

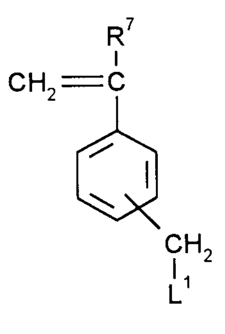
(4)

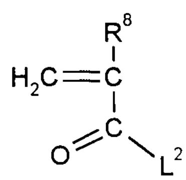
(5)

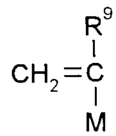
(6)

Die Monomere der Formeln (4) und (5) werden erhalten, indem man eine mit mindestens einer Hydroxy-, Carboxy- und gegebenenfalls Amino-Gruppe funktionalisiertes 1,8-Naphthalimid-Derivat der Formeln (Ia), (Ib) oder (Ic) und ein Styrol- oder Acrylsäure-Derivat der Formeln (7) oder (8)

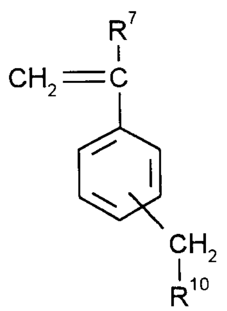
(7)

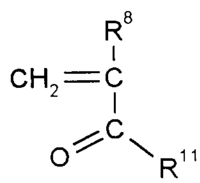
(8)

worin

$R^{10}$ für Halogen, vorzugsweise Chlor oder Brom, steht und

$R^{11}$ für Halogen, vorzugsweise Chlor oder Brom, Hydroxy steht,

in Gegenwart einer Base wie Triethylamin, Pyridin oder ein Alkalimetall-Alkoholat bei Temperaturen von -30°C bis

100°C, vorzugsweise 0°C bis 60°C umsetzt.

Die Monomere der Formel (6) und die Styrol- und Acrylsäure-Derivate der Formeln (7) und (8) sind bekannt oder lassen sich nach bekannten Verfahren herstellen.

Die Polymerisationsverfahren sind in der Literatur beschrieben. Sie können ionisch oder radikalisch erfolgen. Eine anionische Polymerisation kann z.B. durch Starter wie Butyllithium oder Lithiumnaphthalid ausgelöst werden. Eine radikalische Polymerisation kann z.B. durch Radikalstarter wie z.B. Azoinitiatoren oder Peroxide, vorzugsweise AIBN (Azoisobuttersäuredinitril) oder Dibenzoylperoxid, ausgelöst werden. Die Herstellung der Polymere kann in Substanz oder in geeigneten Lösungsmitteln wie Benzol, Toluol, Tetrahydrofuran, Dioxan, Ethylacetat, Xylol, Chlorbenzol, 1-Methoxy-2-propylacetat, chlorierte Kohlenwasserstoffe, Aceton, etc., bei Temperaturen von 20-250°C erfolgen.

Die Verwendung der erfindungsgemäßen 1,8-Naphthalimid-Derivate der Formeln (Ia), (Ib) und (Ic) zur Herstellung von (Co)Polymerisaten mit Luminophoren an den Seitenketten läßt sich beispielsweise durch das erfindungsgemäße Formelschema dargestellen:

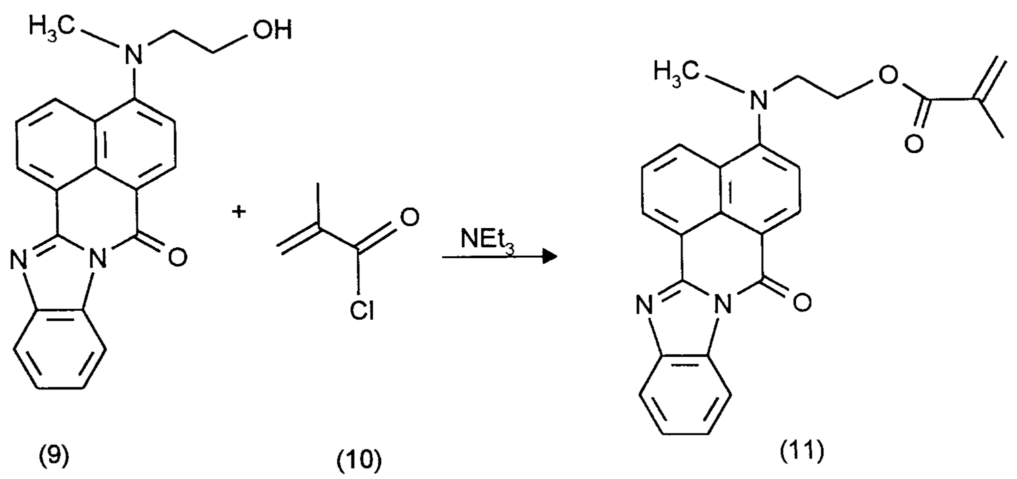


(9) (10) (11)

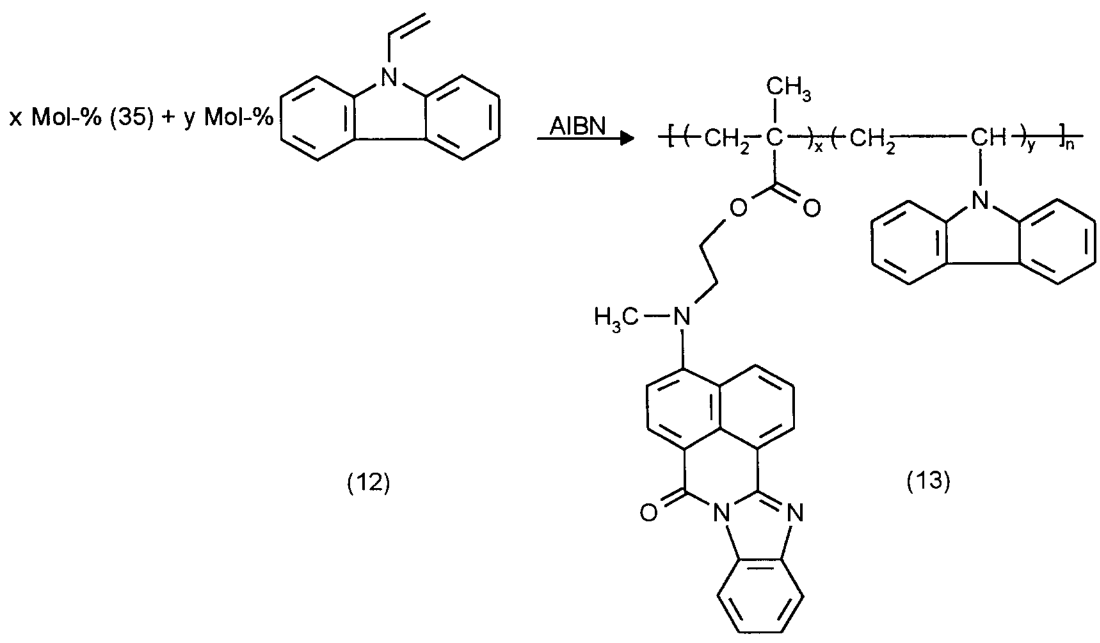


(12) (13)

Hierbei wird ausgehend von 4/5-(N-Methyl-N-hydroxyethyl)amino-1,8-naphthoylen-1'2'-benzimidazol (9) (nur 4-Isomer wird abgebildet) und Methacryloylchlorid (10) unter Mitwirkung von Triethylamin bei 0°C bis Raumtemperatur

zunächst das Methacrylat (11) hergestellt. Das Methacrylat (11) läßt sich in Gegenwart von N-Vinylcarbazol (12) als Comonomer unter Mitwirkung von AIBN als Radikalstarter in Chlorbenzol bei 80°C unter Bildung des Copolymerisats (13) polymerisieren.

Weiterhin kann die Herstellung der Polymerisate oder Copolymerisate beispielsweise durch das folgende Formelschema dargestellt werden:

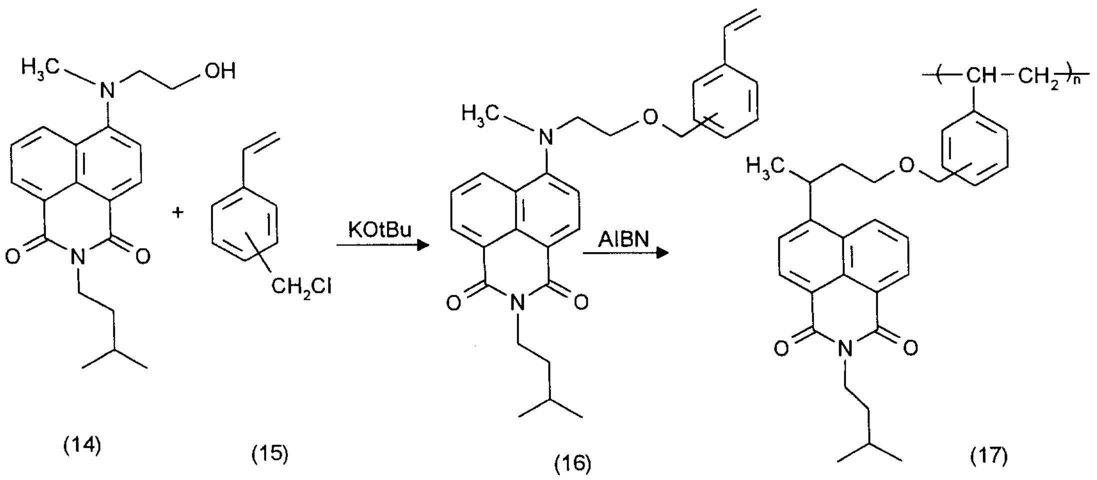


(14) (15) (16) (17)

Hierbei wird ausgehend von N-isoamyl-4-(N'-Methyl-N'-hydroxyethyl)amino-1,8-naphthalimid (14) und m/p-Vinylbenzylchlorid (15) unter Mitwirkung von Kaliumtert-butylat bei Raumtemperatur in Tetrahydrofuran zunächst das Styrolderivat (16) hergestellt. Das Styrolderivat (16) läßt sich unter Mitwirkung von AIBN als Radikalstarter in Toluol bei 100°C unter Bildung des Homopolymerisates (17) polymerisieren.

Die (Co)Polymerisate weisen Molekulargewichte ($\overline{M}w$) im Bereich von 500 bis 1 Million g/Mol, vorzugsweise 800 bis 500.000 g/Mol, bestimmt durch Gelpermeationschromatographie, auf.

Sie zeichnen sich durch ihre lumineszierenden Eigenschaften und Filmbildungsvermögen aus und lassen sich durch Gießen, Rakeln oder Spin-coating auf geeignete Unterlagen aufbringen. Die Produkte zeigen sowohl in Lösungen als auch als Filme Photolumineszenz bei Bestrahlung. Die (Co)Polymerisate sind zum Aufbau von elektrolumineszierenden Anzeigen geeignet, die dadurch gekennzeichnet sind, daß sich eine elektroluminoeszierende Schicht zwischen zwei Elektroden befindet, daß mindestens eine der beiden Elektroden im sichtbaren Spektralbereich transparent ist, daß beim Anlegen einer Gleichspannung im Bereich von 0,1 bis 100 Volt Licht im Frequenzbereich von 200 bis 2000 nm emittiert wird, daß zwischen der elektrolumineszierenden Schicht und den Elektroden zusätzlich eine oder mehrere Zwischenschichten angeordnet sein können.

Diese Zwischenschichten sind aus der Literatur bekannt (vergl. Appl. Phys. Lett., 57, 531 (1990)) und werden dort als HTL (hole transport layer) und als ETL (electron transport layer) bezeichnet. Der Zweck solcher Zwischenschichten liegt u.a. in der Erhöhung der Elektrolumineszenzintensität.

Die erfindungsgemäßen elektrolumineszierenden Polymere können auch als eine Mischung miteinander oder mit mindestens einem weiteren Material in der elektrolumineszierenden Schicht eingesetzt werden. Bei diesem weiteren Material kann es sich um inerte Binder, ladungsträgertransportierende Substanzen wie in EP-A 532 798 oder EP-A 564 224 beschrieben, oder Mischungen aus inerten Bindern und ladungsträgertransportierenden Substanzen handeln.

Die Mischungen aus den erfindungsgemäßen Polymeren und einem weiteren Material zeichnen sich u.a. dadurch aus, daß sie filmbildend sind und durch Gießen, Rakeln oder Spin-coating großflächig auf geeignete Substrate aufgebracht werden können. Als Substrat sind transparente Träger wie Glas oder Kunststoff-Folien (z.B. Polyester, wie Polyethylenterephthalat oder Polyethylennaphthalat, Polycarbonat, Polysulfon, Polyimid-Folien) geeignet.

Bei dem inerten Binder handelt es sich vorzugsweise um lösliche transparente Polymere wie z.B. Polycarbonate, Polystyrol, Polyvinylpyridin, Polymethylphenylsiloxan und Copolymere des Polystyrols wie SAN, Polysulfone, Polyacrylate, Polyvinylcarbazol, Vinylacetat- und Vinylalkoholpolymere und -copolymere.

**Beispiele**

**Beispiel 1:** 4-Chlor-N-hydroxyethyl-1,8-naphthalimid

Ein Gemisch aus 46.6 g (0.20 mol) 4-Chlor-1,8-naphthalsäureanhydrid, 14.6 g (0.24 mol) 2-Aminoethanol und 150 ml N-Methylpyrolidon wird 2 h bei 110°C gerührt. Die Lösung wird auf Raumtemperatur abgekühlt und anschließend mit 1 l Wasser versetzt. Man filtriert die Suspension und erhält nach Trocknen 48 g (87% der Theorie) blaßgelbe Kristalle vom Schmelzpunkt 180-182°C.

**Beispiel 2:** N-Hydroxyethyl-4-piperidino-1,8-naphthalimid

Ein Gemisch aus 20.0 g (0.073 mol) 4-Chlor-N-hydroxyethyl-1,8-naphthalimid, 25.8 g (0.30 mol) Piperidin 2.0 g Kupfer(II)-sulfat und 200 ml Ethylenglykolmonomethylether wird 2 h unter Rückfluß gerührt. Die Lösung wird auf Raumtemperatur abgekühlt und anschließend mit 1 l Wasser versetzt. Man extrahiert die Suspension mit Dichlormethan. Die organische Phase wird eingeengt und der Rückstand aus Toluol umkristallisiert. Man erhält 16 g (68% der Theorie) braune Kristalle vom Schmelzpunkt 152-153°C.

**Beispiel 3:** 4-Chlor-N-isoamyl-1,8-naphthalimid

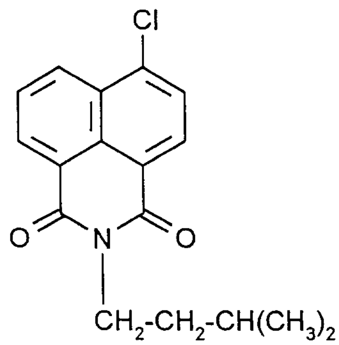

erhält man analog Beispiel 1 aus 4-Chlor-1,8-naphthalsäureanhydrid und Isoamylamin in Essigsäure als Reaktionsmedium in 96%-iger Ausbeute als blaßgelbe Kristalle vom Schmelzpunkt 132.5-134.5°C.

**Beispiel 4:** N-isoamyl-4-(N'-Methyl-N'-hydroxyethyl)amino-1,8-naphthälimid

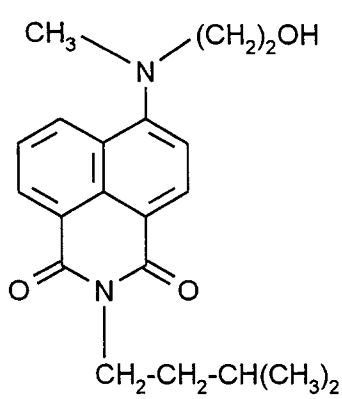

erhält man analog Beispiel 2 aus 4-Chlor-N-isoamyl-1,8-naphthalimid und 2-(Methylamino)-ethanol in 71%-iger Ausbeute als gelbe bis braune Kristalle von Schmelzpunkt 117-118°C.

**Beispiel 5**: N-(5-Carboxypentyl)-4-chlor-1,8-naphthalimid

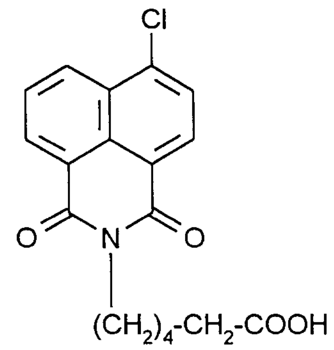

erhält man analog Beispiel 3 aus 4-Chlor-1,8-naphthalsäureanhydrid und 6-Aminohexansäure in 90%-iger Ausbeute als gelbe Kristalle vom Schmelzpunkt 168-171°C.

**Beispiel 6**: N-(5-Carboxypentyl)-4-piperidino-1,8-naphthalimid

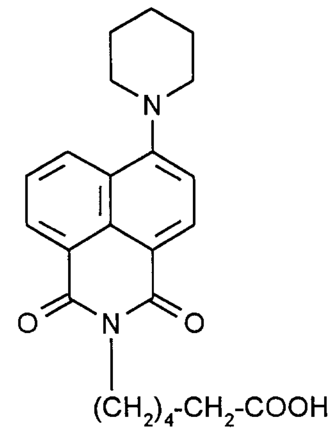

erhält man analog Beispiel 2 aus N-(5-Carboxypentyl)-4-chlor-1,8-naphthalimid und Piperidin in 65%-iger Ausbeute als gelbgrüne Kristalle vom Schmelzpunkt 142-145°C.

**Beispiel 7**: 4/5-Chlor-1,8-naphthoylen-1',2'-benzimidazol

erhält man analog Beispiel 3 aus 4-Chlor-1,8-naphthalsäureanhydrid und o-Phenylendiamin in 87%-iger Ausbeute als gelbe Kristalle vom Schmelzpunkt 225-226°C.

**Beispiel 8**: 4/5-(N-Methyl-N-hydroxyethyl)amino-1,8-naphthoylen-1',2'-benzimidazol

erhält man analog Beispiel 2 aus 4/5-Chlor-1,8-naphthoylen-1',2'-benzimidazol in 82%-iger Ausbeute als rotbraune Kristalle vom Schmelzpunkt 168-169°C.

**Verwendungsbeispiel**

1. Herstellung des N-Isoamyl-4-(N'-Methyl-N'-m/p-vinylbenzyloxyethyl)amino-1,8-naphthalimids gemäß Formel (16)
Zu einer gerührten Lösung von 4,0 g (0,036 Mol) Kalium-tert-butylat in 40 ml trockenem Tetrahydrofuran wird unter

Stickstoff bei 5°C eine Lösung von 10,2 g (0,03 Mol) N-Isoamyl-4-(N'-Methyl-N'-hydroxyethyl)amino-1,8-naphthalimid (Beispiel 4) in 30 ml trockenem Tetrahydrofuran zugetropft. Das Gemisch wird noch 5 h bei Raumtemperatur gerührt. Anschließend wird 5,0 g (0,033 Mol) m/p-Vinylbenzylchlorid zu der rotbraun verfärbten Lösung bei Raumtemperatur zugetropft. Nach 3 h wird die Reaktionsmischung mit 200 ml Wasser und 300 ml Methylenchlorid behandelt. Nach der Phasentrennung wird die wäßrige Phase noch zweimal mit je 100 ml Methylenchlorid extrahiert. Die vereinigten organischen Auszüge werden neutral gewaschen und mit Natriumsulfat getrocknet. Nach Abdestillieren des Lösungsmittels im Vakuum wird der Rückstand durch eine kurze Kieselgelsäule mit Diisopropylether als Laufmittel adsorptiv filtriert, wobei ein Vorlauf mit geringer Menge von m/p-Vinylbenzylchlorid entfernt wird. Nach Abdestillieren des Lösungsmittels erhält man 9,6 g (70 % der Theorie) gelbes Öl.

2. Herstellung des Homopolymerisats gemäß Formel (17)

Eine Lösung von 3,0 g (6,6 mMol) N-Isoamyl-4-(N'-methyl-N'-m/p-vinylbenzyl-oxyethyl)amino-1,8-naphthalimid (16), 15 mg (0,09 mMol) AIBN in 4 ml trockenem Toluol wird unter Vakuum entgast und anschließend unter Stickstoff bei 100°C 3 h gerührt. Die Polymerisationsmischung wird dann mit 15 mg (0,09 mMol) AIBN in drei Portionen innerhalb 3 h nachgestartet. Die Lösung wird anschließend in 100 ml Methanol unter Rühren zugetropft und dann die Suspension abgesaugt. Das Rohprodukt wird noch zweimal aus Methylenchlorid/Methanol-Mischung gefällt. Ausbeute 2,3 g (77 % der Theorie).

3. Herstellung einer elektrolumineszierenden Anordnung

ITO beschichtetes Glas (hergestellt von der Firma Balzers) wird in 20 x 30 mm große Substrate geschnitten und gereinigt. Dabei werden folgende Schritte sukzessiv durchgeführt:

1) 15 min mit dest. Wasser und Falterol im Ultraschallbad spülen,
2) 2 x 15 min mit jeweils frischem dest. Wasser im Ultraschallbad spülen,
3) 15 min mit Ethanol im Ultraschallbad spülen,
4) 2 x 15 min mit jeweils frischem Aceton im Ultraschallbad spülen,
5) Trocknen auf fusselfreien Linsentüchern.

Eine 1 %ige Lösung vom Polymer gemäß Formel (17) in 1,2-Dichlorethan wird gefiltert (0,2 µm-Filter, Firma Sartorius). Die gefilterte Lösung wird mit einer Lackschleuder bei 1000 U/min auf dem ITO-Glas verteilt. Die Dicke des trockenen Films beträgt 110 nm und der Ra-Wert der Oberfläche beträgt 5 nm (Stylusprofilometer Alpha-Step 200 der Firma Tencor Inst.).

Der so hergestellte Film wird anschließend mit Al-Elektroden bedampft. Dazu werden mit Hilfe einer Lochmaske isolierte Al-Punkte mit einem Durchmesser von 3 mm auf den Film gedampft. Während des Aufdampfens herrscht in einer Aufdampfapparatur (Leybold) ein Druck unter $10^{-5}$ mbar.

Die ITO-Schicht und die Al-Elektrode wird über elektrische Zuführung mit einer Spannungsquelle verbunden. Beim Erhöhen der Spannung fließt ein elektrischer Strom durch die Anordnung und die beschriebene Schicht elektrolumineszierts. Die Elektrolumineszenz liegt im gelb-grünen Spektralbereich und tritt bei positiv gepoltem ITO-Kontakt auf.

**Patentansprüche**

1. 1,8-Naphthalimid-Derivate der Formel (Ia), (Ib) und (Ic)

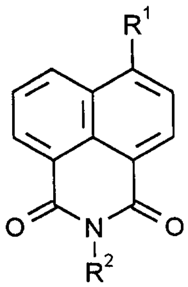 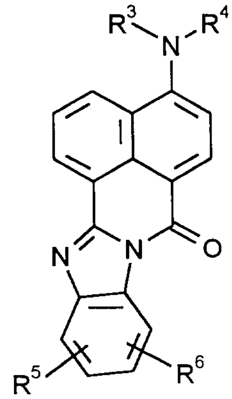 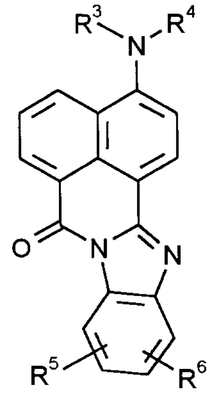

(Ia) (Ib) (Ic)

worin

| | |
|---|---|
| $R^1$ | für Wasserstoff, Halogen, Nitro, $C_1$-$C_4$-Alkoxycarbonyl, $C_1$-$C_4$-Acyl, $C_8$-$C_{24}$-Aralkenyl, unsubstituiertes Amino oder für ein- oder zweifach, gleich oder verschieden durch $C_1$-$C_{30}$-Alkyl, $C_6$-$C_{18}$-Aryl, $C_7$-$C_{24}$-Aralkyl substituiertes Amino steht, wobei die oben genannten Kohlenstoffketten selbst durch Hydroxy und/oder Carboxy substituiert sein können, |
| $R^1$ | weiterhin für Morpholinyl, Piperidinyl, Pyrrolidinyl oder Piperazinyl steht, die einen oder zwei Substituenten ausgewählt aus Hydroxy, Methyl, Ethyl und/oder Phenyl tragen können, |
| $R^2$ | für Wasserstoff oder $C_1$-$C_{30}$-Alkyl, $C_6$-$C_{18}$-Aryl, $C_7$-$C_{24}$-Aralkyl steht, welche ein- oder mehrfach durch Hydroxy und/oder Carboxy substituiert sein können, und mindestens einer der Reste $R^1$ oder $R^2$ über eine Hydroxy- oder Carboxygruppe verfügt, |
| $R^3$ und $R^4$ | unabhängig voneinander für $C_1$-$C_{30}$-Alkyl, $C_6$-$C_{18}$-Aryl, $C_7$-$C_{24}$-Aralkyl stehen, die durch Hydroxy und/oder Carboxy substituiert sein können, wobei mindestens einer der Reste $R^3$ oder $R^4$ über eine Hydroxy- oder Carboxygruppe verfügt, oder |
| $R^3$ und $R^4$ | weiterhin gemeinsam mit dem Stickstoffatom, an das sie gebunden sind, für Morpholinyl, Piperidyl, Pyrolidyl oder Piperazinyl stehen, die einen oder zwei gleich oder verschiedene Substituenten ausgewählt aus Methyl, Ethyl und Phenyl tragen können und über mindestens eine Hydroxy- oder Carboxygruppe verfügen, |
| $R^5$ und $R^6$ | unabhängig voneinander Wasserstoff, Halogen, Nitro, $C_1$-$C_{30}$-Alkyl, $C_1$-$C_{30}$-Alkoxy, $C_6$-$C_{18}$-Aryl, $C_7$-$C_{24}$-Aralkyl, $C_1$-$C_4$-Alkoxycarbonyl, $C_1$-$C_4$-Acyl oder Di($C_1$-$C_6$-Alkyl)amino bedeuten, wobei die aliphatischen Kohlenstoffketten, in $R^1$, $R^2$, $R^3$ und $R^4$ durch eines oder mehrere Heteroatome ausgewählt aus Sauerstoff, Stickstoff und Schwefel und/oder durch einen oder mehrere Phenylenringe, die durch $C_1$-$C_4$-Alkyl und/oder Halogen substituiert sein können, unterbrochen sein |

2. 1,8-Naphthalimid-Derivat gemäß Anspruch 1, worin

| | |
|---|---|
| $R^1$ | für Wasserstoff, Chlor, Brom, Nitro, Methoxycarbonyl, Ethoxycarbonyl, n- oder iso-Propoxycarbonyl, Methylcarbonyl, Ethylcarbonyl, n- oder iso-Propylcarbonyl, Amino, für Amino, welches ein- oder zweifach, gleich oder verschieden durch $C_1$-$C_{15}$-Alkyl, jeweils gegebenenfalls durch Methyl und/oder Ethyl substituiertes Phenyl, Naphthyl, Phenyl-$C_1$-$C_4$-alkyl oder Naphthyl-$C_1$-$C_4$-alkyl substituiert ist, wobei die oben genannten Kohlenstoffketten selbst durch |

Hydroxy und/oder Carboxy substituiert sein können, $R^1$ weiterhin für Morpholinyl, Piperidinyl, Pyrrolidinyl oder Piperazinyl steht, die einen oder zwei Substituenten ausgewählt aus Methyl, Ethyl und/oder Phenyl tragen können,

$R^2$ für $C_1$-$C_{15}$-Alkyl, Phenyl oder Phenyl-$C_1$-$C_6$-alkyl steht, welche durch Hydroxy und/oder Carboxy und die aromatischen Ringen zusätzlich noch durch Halogen, $C_1$-$C_6$-Alkyl oder $C_1$-$C_6$-Alkoxy substituiert sein können, wobei mindestens einer der Reste $R^1$ und $R^2$ über eine Hydroxy- oder Carboxygruppe verfügen muß,

$R^3$ und $R^4$ unabhängig voneinander für $C_1$-$C_{15}$-Alkyl, Phenyl, Phenyl-$C_1$-$C_6$-alkyl, Naphthyl, Naphthyl-$C_1$-$C_6$-alkyl stehen, die einfach oder mehrfach durch Hydroxy oder Carboxy substituiert sein können, wobei mindestens einer der Reste $R^3$ oder $R^4$ über eine Hydroxy- oder Carboxygruppe verfügt oder

$R^3$ und $R^4$ weiterhin gemeinsam mit dem Stickstoffatom, an das sie gebunden sind, für Piperidinyl oder Piperazinyl stehen, die einen oder zwei gleich oder verschiedene Substituenten ausgewählt aus Methyl, Ethyl und Phenyl tragen können und über mindestens eine Hydroxy- oder Carboxygruppe verfügen,

$R^5$ und $R^6$ unabhängig voneinander für Wasserstoff, Halogen, $C_1$-$C_{15}$-Alkyl, $C_1$-$C_{15}$-Alkoxy, $C_1$-$C_4$-Alkoxycarbonyl, $C_1$-$C_4$-Acyl- oder Di-($C_1$-$C_6$-Alkyl)amino, jeweils durch Methyl und/oder Ethyl substituiertes Phenyl, Phenyl-$C_1$-$C_6$-alkyl, Naphthyl oder Naphthyl-$C_1$-$C_6$-alkyl stehen, wobei die aliphatischen Kohlenstoffketten, wie z.B. Alkyl, Alkoxy, Alkylamino, Aralkyl in $R^1$, $R^2$, $R^3$ und $R^4$ durch eines oder mehrere, vorzugsweise ein oder zwei Heteroatome ausgewählt aus Sauerstoff, Stickstoff und Schwefel und/oder durch einen oder mehrere, vorzugsweise ein oder zwei Phenylenringe, die durch $C_1$-$C_4$-Alkyl und/oder Halogen substituiert sein können, unterbrochen sein können.

**3.** 1,8-Naphthalimid-Derivate gemäß Anspruch 1, worin

$R^1$ für Chlor, Brom oder Amino, welches ein- oder zweifach, gleich oder verschieden durch $C_1$-$C_{15}$-Alkyl substituiert für Morpholinyl, Piperidinyl, Pyrrolidinyl oder Piperazinyl steht, wobei die oben genannten Kohlenstoffketten selbst durch Hydroxy oder Carboxy substituiert sein können.

$R^2$ für $C_1$-$C_{12}$-Alkyl, gegebenenfalls durch Halogen, $C_1$-$C_6$-Alkyl, $C_1$-$C_6$-Alkoxy substituiertes Phenyl steht, die eine Hydroxy- oder Carboxygruppe tragen können,

$R^3$ und $R^4$ unabhängig voneinander für $C_1$-$C_{12}$-Alkyl, Phenyl, Phenyl-$C_1$-$C_6$-alkyl stehen, die durch eine Hydroxy- oder Carboxygruppe substituiert sein können und

$R^5$ und $R^6$ unabhängig voneinander für Wasserstoff, Halogen, $C_1$-$C_{12}$-Alkyl, $C_1$-$C_{12}$-Alkoxy, Di($C_1$-$C_6$-alkyl)amino oder Phenyl stehen, wobei mindestens einer der Reste $R^1$ bis $R^4$ über eine Hydroxy- oder Carboxygruppe verfügen muß.

**4.** Verfahren zur Herstellung von 1,8-Naphthalimid-Derivaten der Formel (Ia) gemäß Anspruch 1, wobei man

a) ein 1,8-Naphthalsäureanhydrid der Formel (II) und ein primäres Amin der Formel (III),

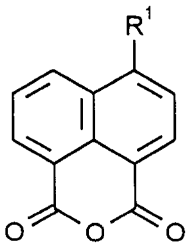

$R^2$—$NH_2$

(II) (III)

bei 50 bis 250°C gegebenenfalls in Gegenwart von Verdünnungsmitteln miteinander umsetzt oder

b) im Falle daß $R^1$ in Formel (Ia) ein- oder zweifach substituiertes Amino oder cyclisches Amino bedeutet, ein 1,8-Naphthalimid der Formel (Id)

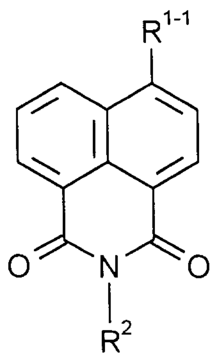

(Id)

worin

$R^{1-1}$ für Halogen oder Nitro steht,

welches aus einem 1,8-Naphthalsäureanhydrid der Formel (IIa) und einem primären Amin der Formel (III)

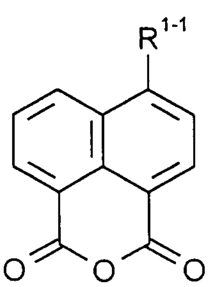

$R^2$-$NH_2$

(IIa) (III)

worin $R^{1-1}$ und $R^2$ die oben genannte Bedeutung haben,
bei Temperaturen von 50 bis 250°C gegebenenfalls in Gegenwart von Verdünnungsmitteln hergestellt wird,

und die so erhaltene Verbindung der Formel (Id) anschließend mit einem primären oder sekundären Amin Piperidin, Morpholin Pyrolidin oder Piperazin gemäß der Definition von $R^1$, oder einer wäßrigen Ammoniaklösung gegebenenfalls in Gegenwart von Lösungsmitteln gegebenenfalls unter Katalyse bei Temperaturen von 50 bis 250°C umsetzt.

**5.** Verfahren zur Herstellung von 1,8-Naphthalimid-Derivaten der Formel (Ib) und (Ic) gemäß Anspruch 1, wobei man ein 1,8-Naphthalimid-Derivat der Formel (IV),

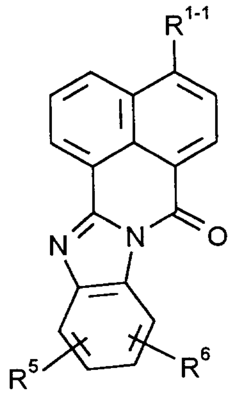

(IVa)

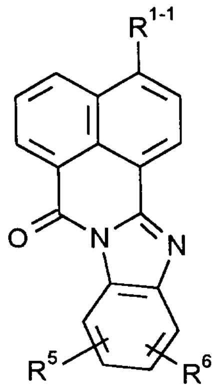

(IVb)

worin
$R^{1-1}$ für Halogen oder Nitro steht,
aus einem 1,8-Naphthalsäureanhydrid der Formel (IIa)

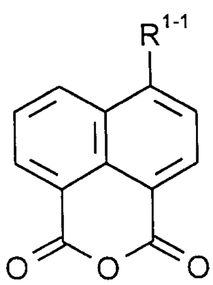

(IIa)

worin $R^{1-1}$ die oben genannte Bedeutung hat,
und einem o-Phenylendiamin der Formel (V)

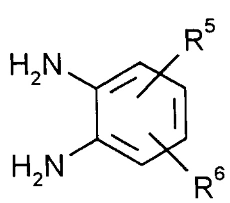

(V)

bei Temperaturen von 50 bis 250°C gegebenenfalls in Gegenwart von Lösungsmitteln herstellt und das 1,8-Naphthalimid-Derivat der Formel (IV) anschließend mit einem sekundären Amin der Formel (VI),

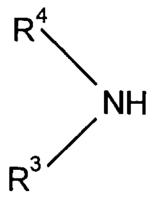

(VI)

unter Katalyse bei Temperaturen von 50 bis 250°C gegebenenfalls in Gegenwart eines Lösungsmittels umsetzt.

6. Verwendung von 1,8-Naphthalimid-Derivaten gemäß Anspruch 1 zur Herstellung von Polymeren mit Luminophoren an den Seitenketten.

Europäisches Patentamt

# EUROPÄISCHER TEILRECHERCHENBERICHT

der nach Regel 45 des Europäischen Patent-übereinkommens für das weitere Verfahren als europäischer Recherchenbericht gilt

Nummer der Anmeldung
EP 96 10 1796

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int.Cl.6) |
|---|---|---|---|
| X | US-A-4 598 081 (EFIMOV) 1.Juli 1986<br>* Anspruch 1 *<br>--- | 1 | C07D221/14<br>C08F255/02<br>C07D471/06<br>//(C07D471/06, 235:00,221:00) |
| X | US-A-4 254 109 (SESTANJ) 3.März 1981<br>* Beispiele 1,2 *<br>--- | 1 | |
| X | US-A-3 821 383 (SESTANJ) 28.Juni 1974<br>* Spalte 8, Zeile 49 - Zeile 53 *<br>--- | 1 | |
| X | EP-A-0 206 322 (I.P.A.) 30.Dezember 1986<br>* Anspruch 1 *<br>--- | 1 | |
| X | CHEMICAL ABSTRACTS, vol. 116, no. 21, 1992<br>Columbus, Ohio, US;<br>abstract no. 210725e,<br>MILLER ET AL.: "Method for determining the binding capacity of albumin in blood serum"<br>Seite 372;<br>XP002004581<br>* Zusammenfassung *<br>& SU-A-1 681 266 (SCIENTIFIC RESEARCH INSTITUTE) 30.September 1991<br>---<br>-/-- | 1 | RECHERCHIERTE SACHGEBIETE (Int.Cl.6)<br>C07D<br>C08F |

## UNVOLLSTÄNDIGE RECHERCHE

**Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.**
**Vollständig recherchierte Patentansprüche:**
**Unvollständig recherchierte Patentansprüche:**
**Nicht recherchierte Patentansprüche:**
**Grund für die Beschränkung der Recherche:**

Siehe Ergänzungsblatt C

| Recherchenort | Abschlußdatum der Recherche | Prüfer |
|---|---|---|
| DEN HAAG | 5.Juni 1996 | Alfaro Faus, I |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C09)

Europäisches Patentamt

# EUROPÄISCHER TEILRECHERCHENBERICHT

Nummer der Anmeldung
EP 96 10 1796

| EINSCHLÄGIGE DOKUMENTE | | | KLASSIFIKATION DER ANMELDUNG (Int.Cl.6) |
|---|---|---|---|
| Kategorie | Kennzeichnung des Dokuments mit Angabe, soweit erforderlich der maßgeblichen Teile | Betrifft Anspruch | |
| X | CHEMICAL ABSTRACTS, vol. 99, no. 3, 1983<br>Columbus, Ohio, US;<br>abstract no. 22909z,<br>EL-NAGGAR: "Synthesis and biological activity of some new 4-bromo-1,8-naphthaloyl amino acids and dipeptide derivatives"<br>Seite 662;<br>XP002004582<br>* Zusammenfassung *<br>& INDIAN J. CHEM., SECT B,<br>Bd. 21B, Nr. 12, 1982,<br>Seiten 1106-09,<br>--- | 1 | |
| X | US-A-5 235 045 (LEWIS) 10.August 1993<br>* Spalte 11, XXI, XXVII; Spalte 12, 46 *<br>--- | 1 | RECHERCHIERTE SACHGEBIETE (Int.Cl.6) |
| X | DE-A-23 60 705 (HOECHST) 26.Juli 1975<br>* Seite 8, Zeile 1 - Zeile 10 *<br>--- | 1 | |
| X | CHEMICAL ABSTRACTS, vol. 117, no. 4, 1992<br>Columbus, Ohio, US;<br>abstract no. 35794y,<br>MALKES ET AL.: "Luminiscent properties of the naphthalic acid derivatives used in defectoscopic liquids"<br>Seite 647;<br>XP002004583<br>Zusammenfassung und Chem. Substance Index, Seite 1507, Spalte 3 (41-52)<br>& ZH. PRIKL. SPEKTROSK.,<br>Bd. 55, Nr. 5, 1991,<br>Seiten 820-25,<br>---<br>-/-- | 1 | |

EPO FORM 1503 03.82 (P04C12)

Europäisches Patentamt

# EUROPÄISCHER TEILRECHERCHENBERICHT

Nummer der Anmeldung
EP 96 10 1796

| EINSCHLÄGIGE DOKUMENTE | | | KLASSIFIKATION DER ANMELDUNG (Int.Cl.6) |
|---|---|---|---|
| Kategorie | Kennzeichnung des Dokuments mit Angabe, soweit erforderlich der maßgeblichen Teile | Betrifft Anspruch | |
| X | CHEMICAL ABSTRACTS, vol. 81, no. 4, 1974<br>Columbus, Ohio, US;<br>abstract no. 154514e,<br>KADHIM ET AL.: "New intermediates and dyes for synthetic polymer fibers."<br>Seite 120;<br>XP002004584<br>* Zusammenfassung und 9th Chem. Subst. Index, Seite 5963, Spalte 3 (60-61); Seite 5968,1(33-35)<br>& J. SOC. DYERS COLOUR. 1974, 90(5), Bd. 90, Nr. 5, 1974, Seiten 153-157,<br>--- | 1 | |
| | | | RECHERCHIERTE SACHGEBIETE (Int.Cl.6) |
| X | POLYMER JOURNAL,<br>Bd. 26, Nr. 4, 1994, TOKYO JP,<br>Seiten 397-402, XP002004580<br>WOO-SIK KIM ET AL.: "Synthesis and fluorescence behaviour of poly[omega-(1,8-naphthalimido)alkyl methacrylates]1"<br>* Verbindungen 1a-1d und 3a-3d *<br>----- | 1,6 | |

EPO FORM 1503 03.82 (P04C12)

EP 96 10 1796

-C-

UNVOLLSTÄNDIGE RECHERCHE

Die Zahl, der im Patentanspruch 1 genannten Verbindungen der Formel Ia ist so gross, dass eine vollständige Recherche aus ökonomischen Gründe nicht möglich ist (siehe Richtlinien für die Prüfung im Europäischen Patentamt, Teil B, Kapitel III.2).